# EUROPEAN PATENT APPLICATION

(11) **EP 4 799 577 A1**
(43) Date of publication of application: **02.09.2026**
(21) Application number: 25811894.2
(22) Date of filing: 20.08.2025
(51) Int. Cl.: A61B 17/064

(54) **SPLIT MODULAR SUTURE ANCHOR IMPLANTATION SYSTEM**

(30) Priority: 05.12.2024 CN 202411774707
(71) Applicant: Star Sports Medicine Co., Ltd., 100176 Beijing (CN)
(72) Inventor: DONG, Wenxing, Beijing 100176 (CN); YIN, Jichen, Beijing 100176 (CN); ZHANG, Zhiliang, Beijing 100176 (CN); YANG, Tengfei, Beijing 100176 (CN)
(74) Representative: Angerhausen, Christoph
(86) International application number: PCT/CN2025/115776
(87) International publication number: WO 2026/118543

(57) **Abstract**

The present application discloses a separable modular suture anchor implant system, relating to the technical field of medical instruments, and including an inserter and a storage box. The inserter includes an insertion rod, a head of which is provided with an insertion portion. The storage box stores an anchor and a suture, an inlet and an outlet are provided at two ends of the storage box respectively, the insertion rod is inserted into the storage box via the inlet, so that the insertion portion is inserted into the anchor, and the anchor is pushed out from the outlet to complete the assembly of the inserter and the anchor; after the anchor is implanted through the inserter, the inserter is moved in a direction opposite to the implantation direction to disconnect the insertion portion from the anchor, and the suture is directly withdrawn without being released by a doctor.

## Description

### CROSS REFERENCE TO RELATED APPLICATION

This application claims priority to Chinese Patent Application No. 2024117747078, filed on December 5, 2024 and entitled "SEPARABLE MODULAR SUTURE ANCHOR IMPLANT SYSTEM", which is hereby incorporated by reference in its entirety.

### TECHNICAL FIELD

The present application relates to the technical field of medical instruments, in particular to a separable modular suture anchor implant system.

### BACKGROUND

Anchors are widely used for the fixation of bones and soft tissues. Soft tissues (such as tendons and ligaments) can be simply and effectively fixed to bones through anchors and sutures around shoulders, knees, hips, elbows, wrists, ankles, etc., without loosening or excessive tension until healing.

In an existing bone anchor implant system, an inserter, an anchor, and a suture pre-assembled into one set are used together during surgery, leading to direct discard of the inserter after surgery. In practice, the quantity and type of anchors are selected according to actual needs, leading to discard of a plurality of inserters in one surgery. The inserters cannot be reused, which not only wastes medical resources and pollutes the environment, but also hinders surgical operations.

### SUMMARY

One of the objectives of the present application is to provide a separable modular suture anchor implant system, which achieves separate storage of anchors and sutures, free assembly of anchors and inserters, and reuse of inserters, thereby reducing medical resource waste and simplifying surgical operations.

To achieve this objective, the present application adopts the following technical solutions.

A separable modular suture anchor implant system includes:
an inserter, including an insertion rod, a head of the insertion rod is provided with an insertion portion; and
a storage box, storing an anchor, an inlet and an outlet are provided at two ends of the storage box respectively, and the insertion rod is inserted into the storage box via the inlet, so that the insertion portion is inserted into the anchor, and the anchor is pushed out from the outlet to complete the assembly of the inserter and the anchor; and after the anchor is implanted through the inserter, the inserter is moved in a direction opposite to the implantation direction to disconnect the insertion portion from the anchor.

As an optional embodiment of the separable modular suture anchor implant system, an anchor clamping assembly is provided in the storage box, the anchor clamping assembly includes an elastic member and two symmetrically provided clamping jaws, the two clamping jaws are connected through the elastic member to form an accommodating chamber, both the inlet and the outlet are in communication with the accommodating chamber, and the anchor is stored in the accommodating chamber; the two clamping jaws are slidably provided in the storage box; and under the push of the insertion rod, the ends of the two clamping jaws close to the outlet slide and open away from each other to push the anchor out of the storage box.

As an optional embodiment of the separable modular suture anchor implant system, a center line of the accommodating chamber coincides with a symmetrical line of the two clamping jaws;
or the center line of the accommodating chamber is perpendicular to the symmetrical line of the two clamping jaws, the two clamping jaws are connected to form a through hole with a center line coinciding with the symmetrical line of the two clamping jaws, and the through hole allows the insertion rod to pass through.

As an optional embodiment of the separable modular suture anchor implant system, a sliding track is provided in the storage box, and the clamping jaw is provided with a sliding column in sliding connection with the sliding track;
each clamping jaw is provided with the sliding columns at two ends on the same side, the sliding track includes a first sliding track and a second sliding track, the first sliding track is provided near the inlet, the second sliding track is provided near the outlet, both the first sliding track and the second sliding track extend away from the clamping jaw and towards the outlet, and the angle between the first sliding track and the symmetric line of the two clamping jaws is smaller than the angle between the second sliding track and the symmetric line of the two clamping jaws;
or each clamping jaw is provided with one sliding column on the same side, and the sliding track extends away from the clamping jaw and towards the outlet.

As an optional embodiment of the separable modular suture anchor implant system, a semi-annular groove is provided along the circumference of the clamping jaw, the semi-circular grooves of the two clamping jaws are docked to form a mounting groove, and the elastic member is provided in the mounting groove;
or the elastic member is provided around the sliding columns provided oppositely on the two clamping jaws.

As an optional embodiment of the separable modular suture anchor implant system, an insertion channel is further provided in the storage box, a center line of the anchor clamping assembly and a center line of the insertion channel are on the same straight line, one end of the insertion channel is in communication with the inlet, and the other end is in communication with the accommodating chamber or the outlet.

As an optional embodiment of the separable modular suture anchor implant system, the storage box further stores a suture, suture storage and winding structures are symmetrically provided on two sides of the anchor, two ends of the suture are connected to the two suture storage and winding structures respectively after the suture passes through the anchor, the insertion rod drives the storage box to move in the direction opposite to the implantation direction, and the suture storage and winding structures release the suture.

As an optional embodiment of the separable modular suture anchor implant system, the suture storage and winding structure includes a ratchet rotatably provided in the storage box; the ratchet includes a central shaft and two baffles provided at two ends of the central shaft, the baffle is provided with spaced suture collection slots, the suture is wound on the central shaft, and a tail end of the suture is clamped into the two adjacent suture collection slots.

As an optional embodiment of the separable modular suture anchor implant system, a plurality of elastic pawls are provided at intervals along the circumference of the baffle, an annular groove is formed between the elastic pawl and the baffle, and one suture collection slot extending radially along the baffle is provided inside each annular groove.

As an optional embodiment of the separable modular suture anchor implant system, a guide damping column is further provided on a peripheral side of the ratchet, and the suture passing through the anchor bypasses the guide damping column and is wound on the central shaft.

As an optional embodiment of the separable modular suture anchor implant system, the suture storage and winding structure includes suture winding slots and suture collection holes, the suture winding slots are symmetrically provided on the periphery of the storage box, one of the suture winding slots is provided with a suture passing hole, and the suture collection holes are symmetrically provided on the periphery of the storage box and are in communication with the interior of the storage box; the suture passing through the anchor passes through the suture passing hole and is wound in the opposite suture winding slot, and the tail end of the suture is clamped into the opposite suture collection hole; and
symmetrical lines of the suture winding slots and the suture collection holes are perpendicular to a line connecting the inlet and the outlet.

As an optional embodiment of the separable modular suture anchor implant system, the suture storage and winding structure further includes a suture storage groove provided in the storage box, the suture is confined within the suture storage groove, and the suture passing through the anchor is led out through the suture storage groove and then passes through the suture passing hole.

As an optional embodiment of the separable modular suture anchor implant system, the suture storage and winding structure further includes a tensioning member provided between the anchor and the suture storage groove, and the suture passing through the anchor is tensioned by the tensioning member and then enters the suture storage groove.

As an optional embodiment of the separable modular suture anchor implant system, the inserter further includes a handle, one end of the handle is provided with a bayonet, the insertion rod extends into the bayonet and is detachably connected to the handle, and the bayonet is used to snap-fit with the storage box.

As an optional embodiment of the separable modular suture anchor implant system, the storage box is provided with a spherical groove, a positioning bead is provided on one side of the bayonet, and the positioning bead cooperates with the spherical groove to fix the inserter and the storage box.

As an optional embodiment of the separable modular suture anchor implant system, a snap-fitting member is provided on one side of the bayonet, and the snap-fitting member is rotatably connected to the handle and cooperates with the wall of the other side of the bayonet to snap-fit with the storage box; and by pressing one end of the snap-fitting member, the other end of the snap-fitting member moves away from the other side wall of the bayonet.

As an optional embodiment of the separable modular suture anchor implant system, the storage box is provided with a plurality of snap-fitting grooves, the other end of the snap-fitting member is provided with a plurality of snap-fitting protrusions, and the snap-fitting protrusions snap-fits with the snap-fitting grooves.

As an optional embodiment of the separable modular suture anchor implant system, the insertion portion includes a prismatic head, an internal hole head, or a fork-shaped head.

As an optional embodiment of the separable modular suture anchor implant system, the insertion portion includes an insertion connection rod, a diameter of the insertion connection rod is smaller than that of the insertion rod, the insertion connection rod is provided with a stress relief platform and scale lines, and the scale lines are provided between the stress relief platform and the insertion rod.

As an optional embodiment of the separable modular suture anchor implant system, the insertion rod is provided with a guide boss.

As an optional embodiment of the separable modular suture anchor implant system, the storage box includes a box body, and the box body includes a top shell and a bottom shell that are engaged.

Beneficial effects of the present application are as follows.

According to the separable modular suture anchor implant system provided in the present application, the anchor and the suture are stored in the storage box separately; the anchor and the suture are assembled to the inserter through the storage box, and during use, only the storage box need to be replaced, while the inserter is not replaced, achieving the purpose of reusing the inserter. Compared with the related art where the anchor and the inserter are assembled one to one and the inserter has to be replaced when the anchor is replaced during use, firstly, the separable modular suture anchor implant system of the present application achieves reuse of the inserter, reduces unnecessary medical waste, saves medical resources, and is beneficial to environmental protection. Secondly, the anchor and the inserter are separated, and the storage box is suitable for storing various anchors. If it is found during surgery that the originally planned anchor size or type is not suitable, material replacement after the adjustment of a surgical plan can be quickly completed by replacing the storage box. Therefore, during surgery, the anchor can be selected by replacing the storage box according to actual surgical needs, which not only simplifies surgical operations but also adapts to complex and variable surgical scenarios. In addition, the anchor and the suture stored in the storage box are assembled with the insertion rod during surgery, avoiding the risk of contamination on the anchor due to exposure during preoperative preparation. Moreover, the anchor snap-fitting assembly in the storage box constrains the freedom of movement of the anchor throughout the entire process, avoiding misalignment, detachment, or jamming of the anchor. Meanwhile, the suture in the storage box can be automatically released through the suture storage and winding structures after being implanted, and its tension can be automatically adjusted during release, so that the suture is released orderly without the risk of entanglement, and manual release of the suture by a doctor or additional adjustment is not required, significantly reducing surgical steps and time.

### BRIEF DESCRIPTION OF THE DRAWINGS

FIG. 1 is a schematic structural diagram before an inserter is assembled with an anchor in a storage box in a separable modular suture anchor implant system according to a first embodiment of the present application;
FIG. 2 is a schematic diagram of a state when an insertion rod of the inserter enters the storage box from an inlet of the storage box according to the first embodiment of the present application;
FIG. 3 is a schematic diagram of a state when the insertion rod pushes out an anchor from an outlet of the storage box according to the first embodiment of the present application;
FIG. 4 is a schematic structural diagram showing that the storage box is snap-fitted with the inserter after the inserter is assembled with the anchor according to the first embodiment of the present application;
FIG. 5 is a cross-sectional view showing that the storage box is snap-fitted with the inserter after the inserter is assembled with the anchor according to the first embodiment of the present application;
FIG. 6 is a cross-sectional view of the inserter according to the first embodiment of the present application;
FIG. 7 is a schematic structural diagram of the storage box according to the first embodiment of the present application;
FIG. 8 is a cross-sectional view of the storage box according to the first embodiment of the present application;
FIG. 9 is an exploded view of the storage box according to the first embodiment of the present application;
FIG. 10 is a schematic diagram of an internal structure of a top shell according to the first embodiment of the present application;
FIG. 11 is a schematic diagram of an internal structure of a bottom shell according to the first embodiment of the present application;
FIG. 12 is a cross-sectional view of an anchor clamping assembly according to the first embodiment of the present application;
FIG. 13 is a schematic structural diagram of a clamping jaw according to the first embodiment of the present application;
FIG. 14 is a schematic diagram when two clamping jaws open after the insertion rod is inserted into the anchor according to the first embodiment of the present application;
FIG. 15 is a schematic structural diagram of a boss anchor according to the first embodiment of the present application;
FIG. 16 is a schematic structural diagram of an internal hole screw according to the first embodiment of the present application;
FIG. 17 is a schematic assembly diagram of the internal hole anchor, a suture, and an insertion portion according to the first embodiment of the present application;
FIG. 18 is a schematic structural diagram of a ratchet according to the first embodiment of the present application;
FIG. 19 is a schematic diagram showing that the suture is wound on the ratchet according to the first embodiment of the present application;
FIG. 20 is a schematic structural diagram of an elastic pawl on a baffle according to the first embodiment of the present application;
FIG. 21 is a cross-sectional view showing that the inserter is fixed to the storage box after pushing the anchor out of the storage box according to the first embodiment of the present application;
FIG. 22 is a schematic diagram of an external structure of the bottom shell according to the first embodiment of the present application;
FIG. 23 is a cross-sectional view of the interior of the storage box when the inserter is not inserted into the storage box according to the first embodiment of the present application;
FIG. 24 is a cross-sectional view when the inserter is inserted into the storage box but not fixed to the anchor according to the first embodiment of the present application;
FIG. 25 is a cross-sectional view when the two clamping jaws open after the inserter is inserted into the storage box and fixed to the anchor according to the first embodiment of the present application;
FIG. 26 is a cross-sectional view when the inserter inserted into the storage box and fixed to the anchor pushes the anchor out of the storage box according to the first embodiment of the present application;
FIG. 27 is a schematic diagram of a state of the suture when the anchor is implanted into the bone by using the separable modular suture anchor implant system according to the first embodiment of the present application;
FIG. 28 is a schematic diagram of a state when a tail end of the suture slides out of suture collection slots after the anchor is implanted into the bone and the inserter is moved in a direction opposite to the implantation direction to disconnect from the anchor according to the first embodiment of the present application;
FIG. 29 is a schematic diagram of a state after the anchor is implanted into the bone and when the inserter moves in the direction opposite to the implantation direction until the tail end of the suture slides out of guide damping columns according to the first embodiment of the present application;
FIG. 30 is a schematic diagram of a state after the anchor is implanted into the bone and when the inserter moves in the direction opposite to the implantation direction until the tail end of the suture slides out of the storage box according to the first embodiment of the present application;
FIG. 31 is a schematic structural diagram showing that the storage box is snap-fitted with the inserter after the inserter is assembled with the anchor according to a second embodiment of the present application;
FIG. 32 is a schematic structural diagram of the storage box according to the second embodiment of the present application;
FIG. 33 is an exploded view of the storage box according to the second embodiment of the present application;
FIG. 34 is a schematic diagram of an internal structure of the bottom shell according to the second embodiment of the present application;
FIG. 35 is a schematic diagram of an internal structure of the top shell according to the second embodiment of the present application;
FIG. 36 is a schematic structural diagram of the inserter according to the second embodiment of the present application;
FIG. 37 is a schematic structural diagram showing that the storage box is snap-fitted with the inserter after the inserter is assembled with the anchor according to a third embodiment of the present application;
FIG. 38 is a cross-sectional view showing that the anchor clamping assembly clamps a full suture anchor according to the third embodiment of the present application;
FIG. 39 is a cross-sectional view showing that the inserter enters the storage box and is inserted into the full suture anchor according to the third embodiment of the present application;
FIG. 40 is a longitudinal cross-sectional view showing that the inserter enters the storage box and is inserted into the full suture anchor according to the third embodiment of the present application;
FIG. 41 is a horizontal cross-sectional view when the two clamping jaws open after the inserter is inserted into the storage box and fixed to the full suture anchor according to the third embodiment of the present application;
FIG. 42 is a horizontal cross-sectional view when the full suture anchor is pushed out of the anchor clamping assembly after the inserter is inserted into the storage box and fixed to the full suture anchor according to the third embodiment of the present application;
FIG. 43 is a horizontal cross-sectional view when the full suture anchor is pushed out of the storage box after the inserter is inserted into the storage box and fixed to the full suture anchor according to the third embodiment of the present application; and
FIG. 44 is a schematic structural diagram of the insertion rod and the insertion portion according to the third embodiment of the present application.

### Reference numerals:

101. Boss anchor; 102. Internal hole anchor; 1021. Crossbar; 103. Full suture anchor;
200. Suture;
1. Inserter; 11. Handle; 111. Bayonet; 112. Positioning bead; 113. Snap-fitting member; 1131. Snap-fitting protrusion; 1132. Pressing portion; 114. Weight reducing cavity; 115. Threaded hole; 12. Insertion rod; 13. Insertion portion; 131. Insertion connection rod; 1311. Fork-shaped head; 1312. Stress relief platform; 1313. Scale line; 14. Guide boss;
2. Storage box; 21. Box body; 211. Top shell; 2111. Hook; 212. Bottom shell; 2121. Spherical groove; 2122. Snap-fitting groove; 213. Insertion channel; 214. Inlet; 215. Outlet; 216. Ratchet groove; 2161. Sleeve; 217. Sliding track; 2171. First sliding track; 2172. Second sliding track; 218. Guide damping column; 2191. Suture winding slot; 2192. Suture collection hole; 2193. Suture passing hole; 2194. Suture storage groove; 2195. Tensioning member; 22. Anchor clamping assembly; 2211. Elastic tape; 2212. Elastic rubber ring; 222. Clamping jaw; 2221. Opening; 2222. Sliding column; 2223. Semi-annular groove; 2224. Limit plane; 2225. Limit inclined plane; 223. Accommodating chamber; 224. Through hole; 23. Ratchet; 231. Central shaft; 232. Baffle; 2321. Suture collection slot; 233. Elastic pawl; 2331. Protruding column.

### DETAILED DESCRIPTION

Embodiments of the present application are described in detail below, and examples of the embodiments are shown in the accompanying drawings. The same or similar reference numerals throughout represent the same or similar elements or elements with the same or similar functions. The embodiments described below with reference to the accompanying drawings are exemplary, and are intended to explain the present application, but shall not be understood as limitations on the present application.

In the description of the present application, it should be noted that the orientations or positional relationships indicated by the terms "center", "upper", "lower", "left", "right", "vertical", "horizontal", "inner", "outer", etc. are based on the orientations or positional relationships shown in the accompanying drawings, and are intended to facilitate the description of the present application and simplify the description only, rather than indicating or implying that the device or element referred to must have a particular orientation or be constructed and operated in a particular orientation. Therefore, these terms cannot be interpreted as limiting the present application. In addition, the terms "first" and "second" are merely for description, and cannot be interpreted as indicating or implying relative importance. The terms "first position" and "second position" refer to two different positions.

Unless otherwise specified and limited, the terms "installed", "connected", "connection", and "fixed" should be understood in a broad sense. For example, the "connection" may be fixed connection, detachable connection, mechanical connection, electrical connection, direct connection, indirect connection by a medium, internal communication of two elements, or interaction between two elements. A person of ordinary skill in the art may understand the specific meanings of the above terms in the present application according to specific circumstances.

Unless otherwise specified and limited, the first feature "above" or "below" the second feature may include direct contact between the first feature and the second feature, or may include contact between the first feature and the second feature through another feature between them instead of direct contact. Moreover, the first feature "on", "above", and "up" the second feature may include the first feature right above or obliquely above the second feature, or merely indicates that the level of the first feature is higher than that of the second feature. The first feature "below", "under", and "down" the second feature may include the first feature right below or obliquely below the second feature, or merely indicates that the level of the first feature is lower than that of the second feature.

The technical solution of the present application will be further explained through specific implementations in conjunction with the accompanying drawings.

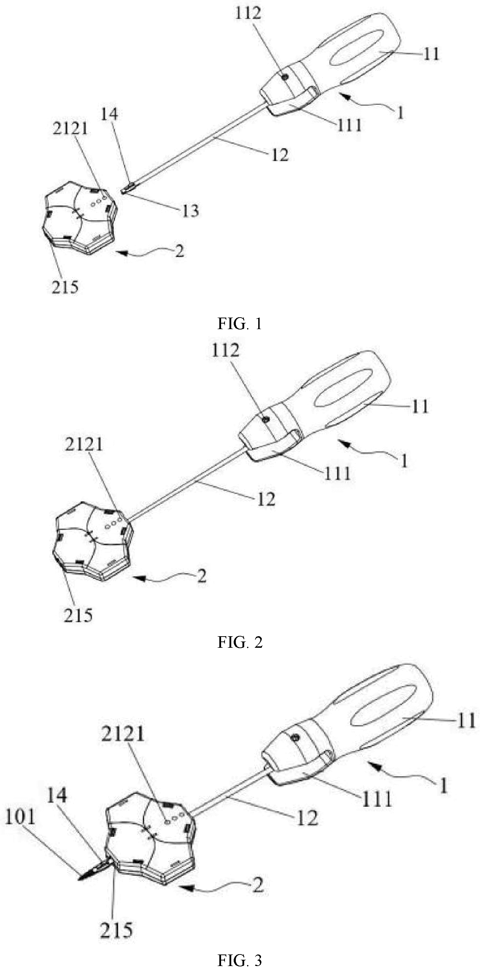

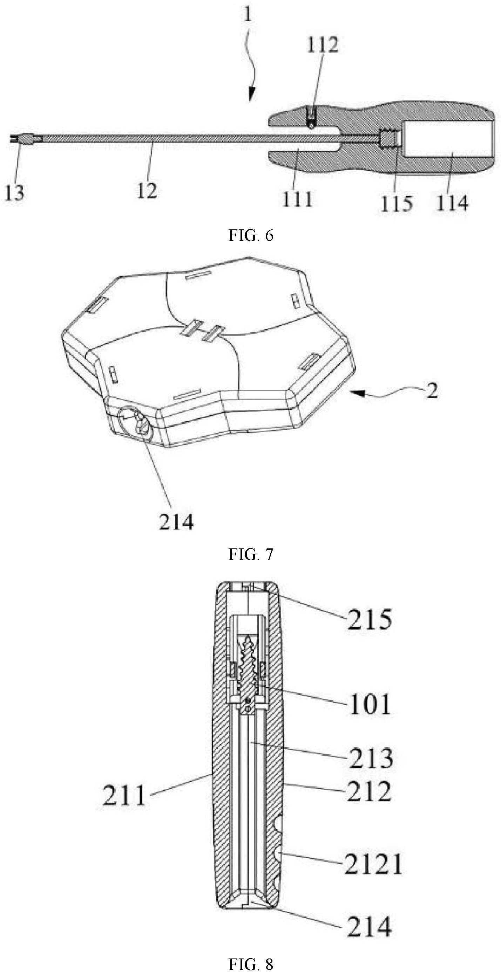

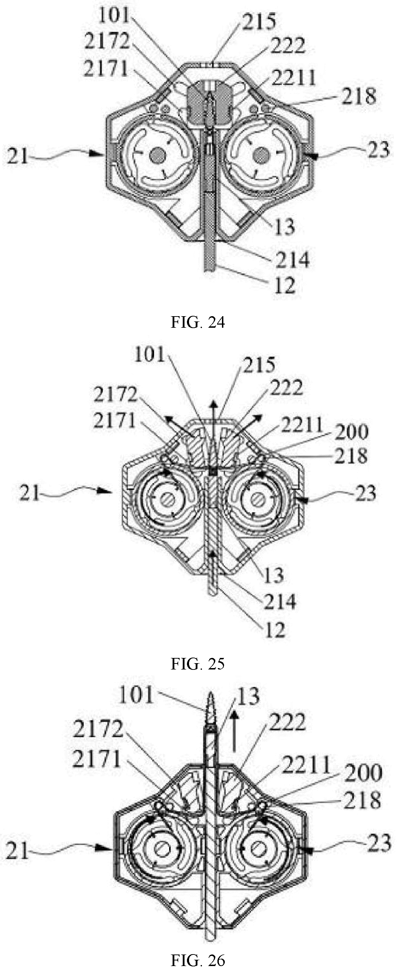

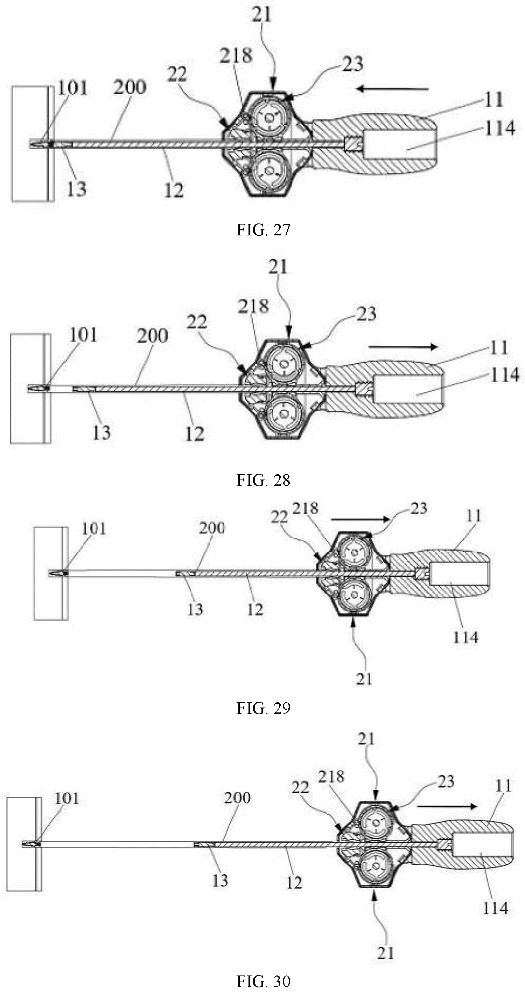

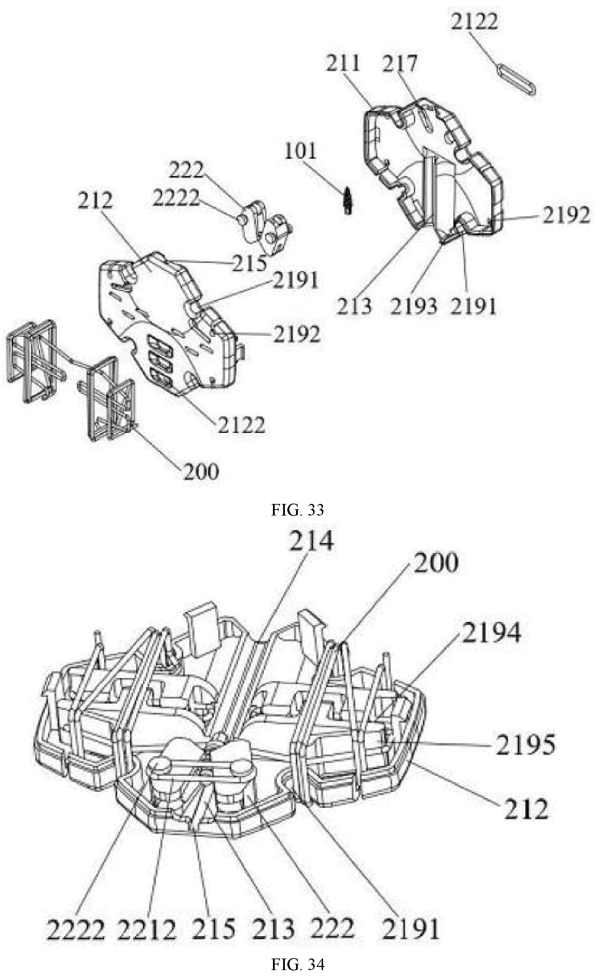

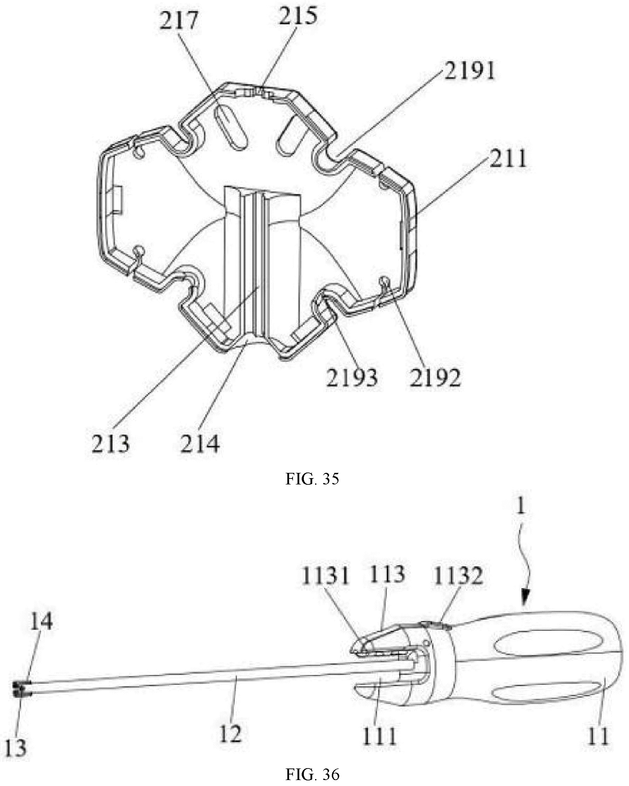

As shown in FIGS. 1 to 44, embodiments of the present application provide a separable modular suture anchor implant system, including an inserter 1 and a storage box 2. The storage box 2 stores an anchor and a suture 200. The anchor and the suture 200 may be stored in one storage box 2 or stored in different storage boxes 2 respectively. During implant surgery, the inserter 1 is inserted into the storage box 2 to assemble with the anchor. After the inserter 1 pushes the anchor out of the storage box 2, the inserter 1 is fixed to the storage box 2. After the inserter 1 is assembled with the anchor, the anchor is implanted into the bone by using the inserter 1, and a force is applied to the inserter 1 in a direction opposite to the implantation direction. After the inserter 1 is disconnected from the anchor, the suture 200 is released. Therefore, surgical operations are simplified, and surgical efficiency is improved. Finally, the storage box 2 is removed from the inserter 1. The inserter 1 may be reused by replacing the storage box 2, thereby solving the problems of resource waste, environmental pollution, etc. caused by joint use of the anchor and the inserter 1 and the disposable attribute of the inserter 1 in the related art. Meanwhile, the reusable inserter 1 saves costs, ensures structural strength, and reduces the frequency of instrument failure during surgery due to the quality of the inserter 1. The anchor and the suture 200 are stored in the storage box 2 separately to avoid environmental contamination on the anchor exposed during preoperative preparation, thereby reducing surgical risks. Moreover, the volume of the storage box 2 is relatively small, allowing to prepare enough and various storage boxes 2 before surgery, so as to ensure emergency response during surgery.

The separable modular suture anchor implant system provided in the present application is suitable for the implantation of anchors of various materials and shapes. For example, according to different materials, the anchors include metal anchors, polyetheretherketone anchors, absorbable anchors, full suture anchors 103, and interface anchors; and according to different shapes, the anchors include boss anchors 101 and internal hole anchors 102. In addition, the shape of the full suture anchors 103 is different from that of the boss anchors 101 and the internal hole anchors 102.

The inserter 1 includes a handle 11 and an insertion rod 12, one end of the handle 11 is provided with a bayonet 111, the insertion rod 12 extends into the bayonet 111 and is detachably connected to the handle 11, and the bayonet 111 is used to snap-fit with the storage box 2. A force is applied to the insertion rod 12 through the handle 11 to insert the insertion rod 12 into the storage box 2 and push out the anchor, and a force is applied to the anchor through the handle 11 in the process of anchor implantation. A head of the insertion rod 12 is provided with an insertion portion 13, and the insertion portion 13 is inserted into the anchor. Different insertion portions 13 are adapted to the anchors of different materials and shapes.

For example, for a full suture anchor 103 or a percussion implant anchor, the tail of the handle 11 is percussed with a hammer to percuss the anchor into a predetermined position and depth, and the percussion immediately stops. Then, the handle 11 of the inserter 1 is held and pulled out in the direction opposite to the implantation direction, and the storage box 2 releases the suture 200. When the suture 200 is completely released out of the storage box 2, the implantation is completed.

An end of the insertion rod 12 away from the insertion portion is provided with an external thread, and the bottom center of the bayonet 111 is provided with a threaded hole 115. The insertion rod 12 extends into the threaded hole 115 and is screwed into the handle 11, facilitating quick replacement of the insertion rod 12. For anchors of different materials and shapes, different types and specifications of insertion rods 12 may be replaced. The insertion portion 13 is connected to the insertion rod 12 by welding, with guiding and fitting functions, while ensuring the reliability of connection between the insertion portion 13 and the insertion rod 12, thereby avoiding medical accidents where the insertion portion 13 remains in the bone due to the disconnection of the insertion portion 13 from the insertion rod 12 when a force is applied in the direction opposite to the implantation direction to disconnect the inserter 1 from the anchor.

Further, a weight reducing cavity 114 is provided at the end of the handle 11 away from the bayonet 111 to reduce the overall weight of the inserter 1.

Since the inserter 1 is a reusable surgical tool, the costs of key parts of the inserter 1 can be appropriately increased to ensure quality and improve mechanical stability. The insertion rod 12 and the insertion portion 13 made of hard stainless steel have much higher strength than the insertion rod 12 made of disposable stainless steel in the related art, thereby effectively avoiding material failures of the insertion rod 12, such as thread slippage, bending, torsional deformation, and fatigue fracture. The handle 11 with moderate weight avoids operational errors caused by excessively light plastic handles in the related art.

### First embodiment:

As shown in FIGS. 1 to 9, this embodiment provides a separable modular suture anchor implant system. An anchor is stored in a storage box 2, and an inlet 214 and an outlet 215 are provided at two ends of the storage box 2 respectively. An insertion rod 12 can be inserted into the storage box 2 via the inlet 214, so that an insertion portion 13 is in insertion fit with the anchor, and the anchor is pushed out from the outlet 215 to complete the assembly of an inserter 1 and the anchor. After the anchor is implanted through the inserter 1, the inserter is moved in a direction opposite to the implantation direction to disconnect the insertion portion 13 from the anchor.

Specifically, an insertion channel 213 is provided in the storage box 2, and at least a portion of the anchor corresponds to the center line of the insertion channel 213, so that the insertion rod 12 enters the storage box 2 along the insertion channel 213. The insertion channel 213 guides the insertion rod 12, and the insertion portion 13 is inserted and fixed to the anchor. After the insertion rod 12 pushes the anchor out of the storage box 2, the insertion rod 12 moves to a set position, and the inserter 1 is fixed to the storage box 2 to prevent the movement of the storage box 2 on the insertion rod 12 from affecting the implantation of the anchor when the anchor is implanted through the inserter 1.

In other embodiments, the insertion rod 12 may be directly in insertion fit with the anchor after entering the storage box 2 via the inlet 214. In this case, the insertion channel 213 may not be provided in the storage box 2.

The inserter 1 of the separable modular suture anchor implant system may be reused by replacing the storage box 2, thereby reducing costs and the probability of mechanical failure; the anchor is stored separately, which can avoid environmental contamination on the implant during preoperative preparation during implantation surgery and reduce surgical risks; meanwhile, the small volume of the storage box 2 facilitates preoperative material preparation.

The storage box 2 includes a box body 21, an anchor clamping assembly 22, and a suture storage and winding structure. The anchor clamping assembly 22 is provided in the box body 21 to clamp the anchor. After the anchor is fixed to the insertion portion 13, under the pushing force of the inserter 1, the anchor can break from the clamping force of the anchor clamping assembly 22 and be pushed out from the anchor clamping assembly 22. The anchor is provided with a threading hole, and two ends of a suture 200 are stored in the suture storage and winding structure after the suture passes through the threading hole. When the anchor is pushed out of the box body 21 by the inserter 1, the suture 200 is pulled, the suture storage and winding structure begins to release the suture 200 until the anchor is implanted into the bone, and the inserter 1 moves in the direction opposite to the implantation direction, enabling the suture 200 to slide out of the box body 21 and be completely released.

In this embodiment, the anchor and the suture 200 are stored in the same storage box 2. In other embodiments, the anchor and the suture 200 may be stored separately.

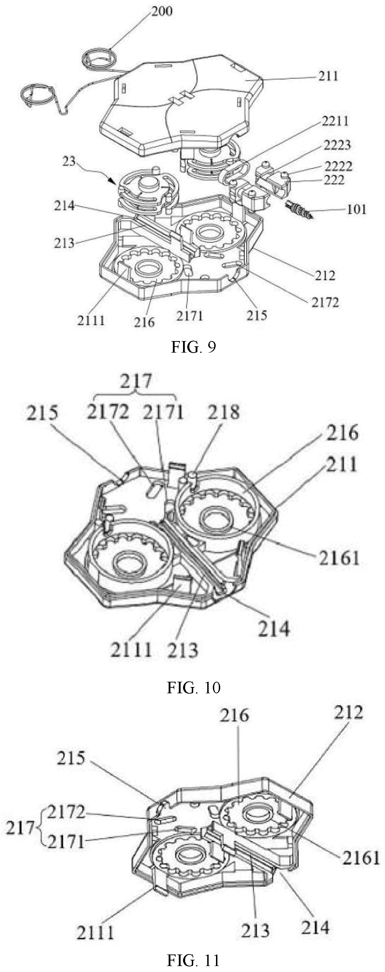

As shown in FIGS. 9 to 11, the box body 21 includes a top shell 211 and a bottom shell 212 that are snap-fitted. The top shell 211 and the bottom shell 212 are fastened and snap-fitted. The top shell 211 and the bottom shell 212 are snap-fitted through the cooperation of a hook 2111 and a hook groove. The top shell 211 and the bottom shell 212 are fastened and snap-fitted to form a storage space therein, and two opposite ends of the storage space are provided with the inlet 214 and the outlet 215 respectively. The anchor and the suture 200 are stored in the storage space, and the insertion channel 213 extends along a line connecting the inlet 214 and the outlet 215. Both the inlet 214 and the outlet 215 can be in communication with the insertion channel 213. The insertion rod 12 enters the storage space from the inlet 214, moves along the insertion channel 213 to be inserted into the anchor, and pushes out the anchor from the outlet 215.

Specifically, two raised ribs are provided about a central symmetric line of each of the top shell 211 and the bottom shell 212, and the two raised ribs on the top shell 211 are docked with the two raised ribs on the bottom shell 212 to form the insertion channel 213. A center line of the anchor clamping assembly 22 and a center line of the insertion channel 213 are on the same straight line, so that when the insertion rod 12 enters the insertion channel 213 via the inlet 214, the insertion channel 213 provides guidance for the insertion rod 12. Under the guidance of the insertion channel 213, the insertion rod 12 can move to the anchor clamping assembly 22, ensuring that the insertion portion 13 on the insertion rod 12 can be inserted and fixed to the anchor clamped in the anchor clamping assembly 22, and then the anchor can be pushed out of the box body 21. Two suture storage and winding structure are designed and provided on two sides of the anchor respectively. By such arrangement, the two ends of the suture 200 passing through the anchor are stored and released through the two suture storage and winding structures.

The outer contour of the box body 21 is approximately cross, with two wing areas used for accommodating the suture storage and winding structures, and head and tail areas used for accommodating the insertion channel 213 and the anchor clamping assembly 22 respectively.

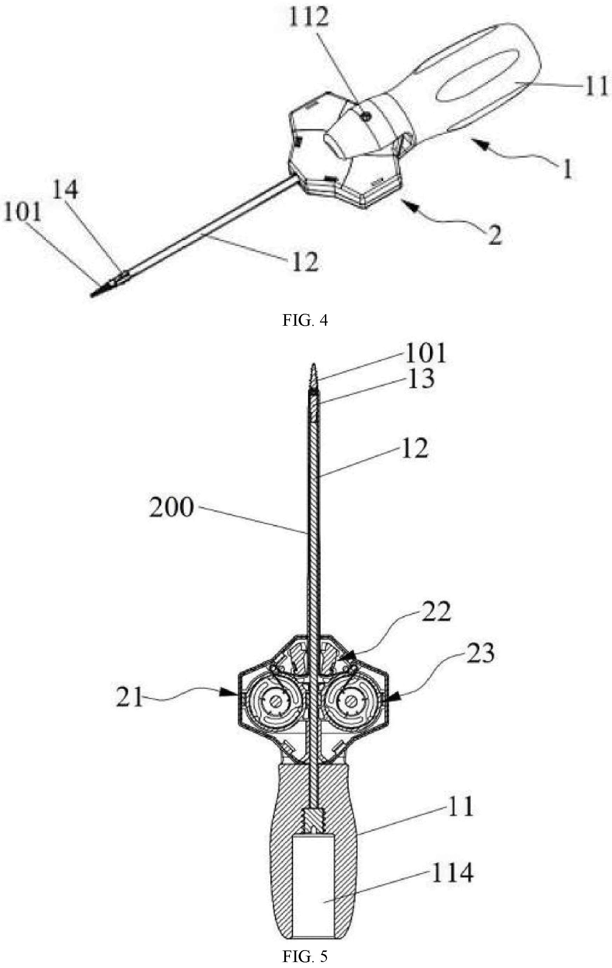

With continued reference to FIGS. 1 and 5, the insertion rod 12 further includes a guide boss 14 that cooperates with the insertion channel 213. The diameter of the insertion rod 12 is smaller than the inner diameter of the insertion channel 213. When the insertion rod 12 enters the box body 21, the guide boss 14 cooperates with the insertion channel 213 to provide guidance for the movement of the insertion rod 12. The guide boss 14 may be provided at the end of the insertion rod 12 near the insertion portion 13.

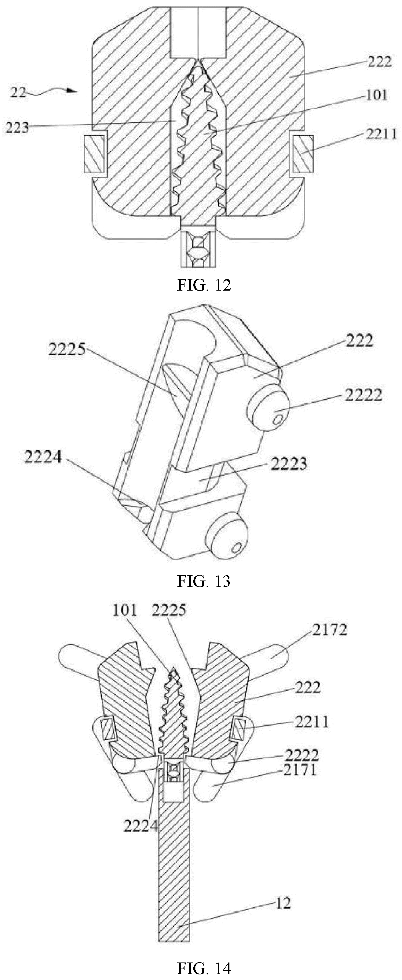

As shown in FIGS. 12 to 14, specifically, the anchor clamping assembly 22 includes an elastic member and two symmetrically provided clamping jaws 222, the two clamping jaws 222 are connected through the elastic member to form an accommodating chamber 223, both the inlet 214 and the outlet 215 are in communication with the accommodating chamber 223, and the anchor is stored in the accommodating chamber 223; the two clamping jaws 222 are slidably provided in the storage box 2; and under the push of the insertion rod 12, the ends of the two clamping jaws 222 near the outlet 215 of the storage box 2 slide and open away from each other to push the anchor out of the storage box 2. The two clamping jaws 222 are connected through the elastic member, so that the accommodating chamber 223 formed by the two clamping jaws 222 under the action of the elastic member can clamp the anchor; the two clamping jaws 222 are slidably provided in the storage box 2, so that the anchor can drive the two clamping jaws 222 to move away from each other under the push of the insertion rod 12; and the ends of the two clamping jaws 222 near the outlet 215 open, so that the insertion rod 12 pushes the anchor out of the accommodating chamber 223.

In this embodiment, one end of the insertion channel 213 is in communication with the inlet 214, and the other end is in communication with the accommodating chamber 223. That is, the insertion channel 213 extends from the inlet 214 to the accommodating chamber 223. When the insertion channel 213 extends from the inlet 214 to the accommodating chamber 223, the raised ribs of the insertion channel 213 do not provide constraint near the outlet 215, which facilitates the disassembly of the box body 21 from the inserter 1; and the anchor clamping assembly 22 is interfered, which can increase the volume of the accommodating chamber 223 formed by the two clamping jaws 222 and adapt to the clamping of anchors with larger sizes.

A center line of the accommodating chamber 223 coincides with a symmetrical line of the two clamping jaws 222, that is, the anchor is placed in the accommodating chamber 223, and the axis of the anchor is located on the symmetrical line of the two clamping jaws 222. The insertion rod 12 moves along the insertion channel 213 until the insertion portion 13 is inserted into the tail of the anchor. Such arrangement is suitable for clamping a boss anchor 101 or an internal hole anchor 102.

The insertion portion 13 is designed according to the shape of the anchor, including a prismatic head or an internal hole head.

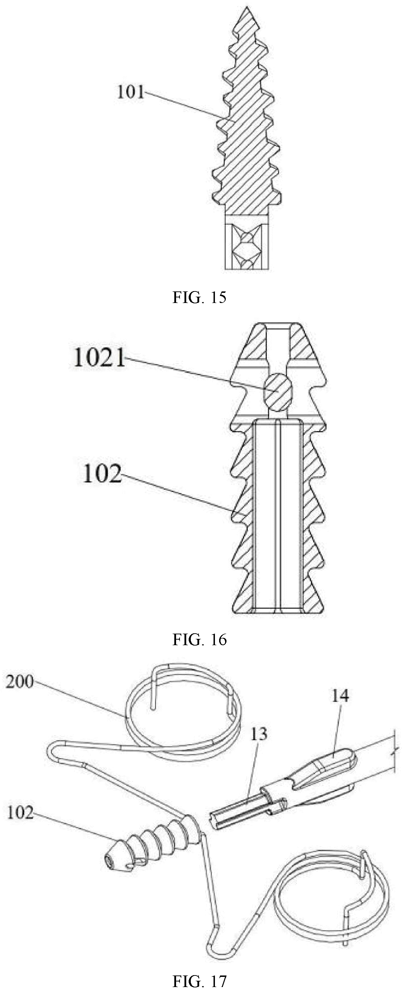

For example, as shown in FIG. 15, the tail of the boss anchor 101 is in a hexagonal pyramid shape, and the insertion portion 13 is designed as an internal hole head, that is, a hexagonal hole matching the hexagonal pyramid. The threading hole of the boss anchor 101 penetrates two opposite sides of the hexagonal pyramid. Meanwhile, in order to facilitate the connection between the suture 200 passing through the threading hole and the suture storage and winding structure, two opposite sides of the hexagonal hole are in communication with each other.

As shown in FIGS. 16 and 17, the internal hole of the internal hole anchor 102 is a hexagonal hole, the hexagonal hole extends from the tail to head of the internal hole anchor 102, a crossbar 1021 is provided in the hexagonal hole near the head of the internal hole anchor 102, and one end of the suture 200 enters from the hexagonal hole, passes through the crossbar 1021, and then passes back. The insertion portion 13 is designed as a prismatic head, that is, designed as a hexagonal prism matching the hexagonal hole. Two opposite sides of the hexagonal prism are provided with clearance slots, and the two ends of the suture 200 pass through the clearance slots and extend out from the tail of the internal hole anchor 102.

For the formation of the accommodating chamber 223, with continued reference to FIGS. 12 and 13, a cavity is provided on each of the close sides of the two clamping jaws 222, and the two cavities are connected to form the accommodating chamber 223. A limit plane 2224 is provided at the end of the cavity near the inlet 214, and a limit inclined plane 2225 is provided at the end of the cavity near the outlet 215. The limit plane 2224 and the limit inclined plane 2225 jointly limit the movement of the anchor in the accommodating chamber 223, ensuring that the insertion portion 13 can be smoothly inserted into the anchor.

Further, the cavity is designed in the middle of the clamping jaw 222, and a semi-circular hole is provided at each of two ends of the cavity. After the two clamping jaws 222 are docked, the opposite semi-circular holes of the two clamping jaws 222 are docked to form clearance holes allowing the insertion rod 12 to pass through.

A semi-annular groove 2223 is provided along the circumference of the clamping jaw 222, the semi-circular grooves 2223 of the two clamping jaws 222 are docked to form a mounting groove, and the elastic member is provided in the mounting groove. The elastic member is designed as an elastic tape 2211 adapted to the mounting groove, and the two clamping jaws 222 are oppositely fixed together by the elastic force of the elastic tape 2211 to store the anchor. Meanwhile, the insertion rod 12, after inserted and fixed to the anchor, applies a larger pushing force to the anchor, and the anchor drives the two clamping jaws 222 to overcome the elastic force of the elastic tape 2211, so that the ends of the two clamping jaws 222 near the outlet 215 move away from each other, the end of the anchor clamping assembly 22 near the outlet 215 opens, and the anchor is pushed out of the accommodating chamber 223 and slides out from the outlet 215.

With continued reference to FIGS. 10 and 13, a sliding track 217 is provided in the storage box 2, and the clamping jaw 222 is provided with a sliding column 2222 in sliding connection with the sliding track 217. When the insertion rod 12 pushes the anchor, the anchor applies a pushing force to the two clamping jaws 222. When the two clamping jaws 222 overcome the elastic force of the elastic tape 2211 and move away from each other, the sliding columns 2222 cooperate with the sliding tracks 217 to guide the movement direction of the two clamping jaws 222. After the anchor is pushed out of the accommodating chamber 223, the two clamping jaws 222 can recover along the sliding tracks 217 under the elastic restoring force of the elastic tape 2211.

Each clamping jaw 222 is provided with the sliding columns 2222 at two ends on the same side, the sliding track 217 includes a first sliding track 2171 and a second sliding track 2172, the first sliding track 2171 is provided near the inlet 214, the second sliding track 2172 is provided near the outlet 215, both the first sliding track 2171 and the second sliding track 2172 extend away from the clamping jaw 222 and towards the outlet 215, and the angle between the first sliding track 2171 and the symmetric line of the two clamping jaws 222 is smaller than the angle between the second sliding track 2172 and the symmetric line of the two clamping jaws 222. When the insertion rod 12 pushes the anchor, the anchor applies a force to the ends of the clamping jaws 222 near the outlet 215, the two clamping jaws 222 open at the end near the outlet 215, and the opening angle of the end near the inlet 214 is smaller than that of the end near the outlet 215. Therefore, the angle between the first sliding track 2171 and the symmetric line of the two clamping jaws 222 is smaller than the angle between the second sliding track 2172 and the symmetric line of the two clamping jaws 222. Through the two sliding tracks 217, the movement path of the clamping jaws 222 can be better controlled to ensure the opening angle of the clamping jaws 222 at the end near the outlet 215.

Specifically, with continued reference to FIGS. 9 to 11, both the top shell 211 and the bottom shell 212 are provided with the first sliding track 2171 and the second sliding track 2172, and two sliding columns 2222 are provided on two sides of the clamping jaw 222. In this way, the two sliding columns 2222 on the side of the clamping jaw 222 near the top shell 211 are in sliding fit with the first sliding track 2171 and the second sliding track 2172 correspondingly provided on the top shell 211, and the two sliding columns 2222 on the side of the clamping jaw 222 near the bottom shell 212 are in sliding fit with the first sliding track 2171 and the second sliding track 2172 correspondingly provided on the bottom shell 212 respectively, to ensure the stability and reliability of movement of the two clamping jaws 222.

The suture storage and winding structures are symmetrically provided on two sides of the anchor. Two ends of the suture 200 are connected to the two suture storage and winding structures respectively after the suture passes through the anchor, the insertion rod 12 drives the storage box 2 to move in the direction opposite to the implantation direction, and the suture storage and winding structures can automatically release the suture 200. Before surgery, the suture 200 is stored in the two suture storage and winding structures after passing through the two ends of the anchor. After the anchor is implanted, the entire suture 200 can be released by pulling the handle 11 in the direction opposite to the implantation direction, and the suture 200 does not need to be manually released, thereby further simplifying surgical operations and improving surgical efficiency.

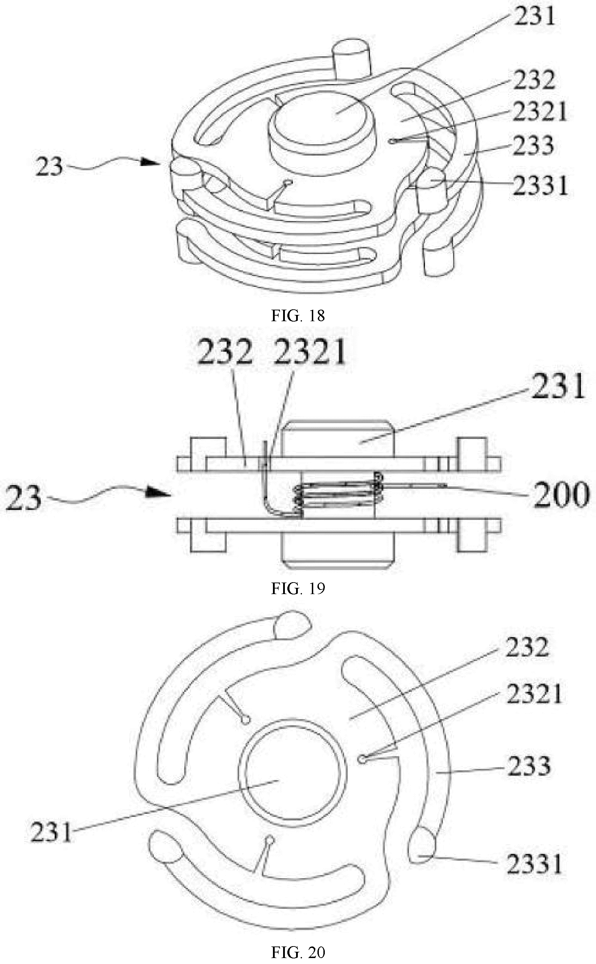

Specifically, as shown in FIGS. 18 to 20, the suture storage and winding structure includes a ratchet 23, and the ratchet 23 is rotatably provided in the storage box 2; the ratchet 23 includes a central shaft 231 and two baffles 232 provided at two ends of the central shaft 231, the baffle 232 is provided with spaced suture collection slots 2321, the suture 200 is wound on the central shaft 231, and a tail end of the suture 200 is clamped into the two adjacent suture collection slots 2321. The suture 200 is wound on the central shaft 231 for storage, and the tail end of the suture 200 is snap-fitted into the two adjacent suture collection slots 2321 for fixation to prevent loosening. When the suture 200 is released, under the tension of the anchor, the tail end of the suture 200 can automatically break from the suture collection slots 2321 without manual release.

The ratchet 23 may carry various types of sutures 200, such as flat ribbon sutures, flat round sutures, absorbable sutures, barbed sutures, coated sutures, and sandwich sutures. In addition, the ratchet 23 has enough space to accommodate various sizes of sutures 200, such as 6-0, 4-0, 2-0, #2, #4, and #6.

Specifically, with continued reference to FIGS. 10 and 11, both the top shell 211 and the bottom shell 212 are provided with ratchet grooves 216, the ratchet 23 is provided in an accommodating space formed by the corresponding ratchet grooves 216 of the top shell 211 and the bottom shell 212, a sleeve 2161 in rotating fit with the central shaft 231 is provided in the center of the ratchet groove 216, two ends of the central shaft 231 are provided in the sleeve 2161 respectively, and when the suture 200 is pulled by the anchor, the central shaft 231 rotates relative to the sleeves 2161 to gradually release the suture 200.

Further, as shown in FIG. 20, a plurality of elastic pawls 233 are provided at intervals along the circumference of the baffle 232, an annular groove is formed between the elastic pawl 233 and the baffle 232, and one suture collection slot 2321 extending radially along the baffle 232 is provided inside each annular groove. A protruding column 2331 is provided at an end of the elastic pawl 233. During the rotation of the ratchet 23, the protruding column 2331 is in contact with the inner wall of the ratchet groove 216 to damp the pulling-out of the suture 200, ensuring that the suture 200 can be stably pulled out. The plurality of elastic pawls 233 provide stable torque for the pulling-out of the suture 200, ensuring the symmetry of the torque received by the central shaft 231. In addition, during the rotation of the ratchet 23, the protruding column 2331 touches the ratchet groove 216 to produce a clicking sound. The sound provides a reminder for medical staff that the suture 200 is pulled out normally.

With continued reference to FIGS. 10 and 11, a guide damping column 218 is further provided on a peripheral side of the ratchet 23, and the suture 200 passing through the anchor bypasses the guide damping column 218 and is wound on the central shaft 231. The suture 200 bypasses the guide damping column 218, and the guide damping columns 218 can provide damping for the pulling and stretching of the suture 200, thereby reducing low-frequency vibration generated at the gap between the elastic pawls 233 on the ratchet 23, and ensuring uniform pulling force for the suture 200 during the release of the suture 200.

Specifically, a notch is provided in the circumferential direction of the ratchet groove 216, the guide damping column 218 is provided outside the notch, and two guide damping columns 218 are provided, with one provided on one side of the notch near the clamping jaw 222, and the other provided opposite to the notch away from the clamping jaw 222. The suture 200 passing through the anchor first bypasses the guide damping column 218 near the clamping jaw 222, then surrounds the guide damping column 218 away from the clamping jaw 222, enters the ratchet groove 216 through the notch, and is wound on the central shaft 231. Such arrangement enables the suture 200 to maintain tension throughout the release process.

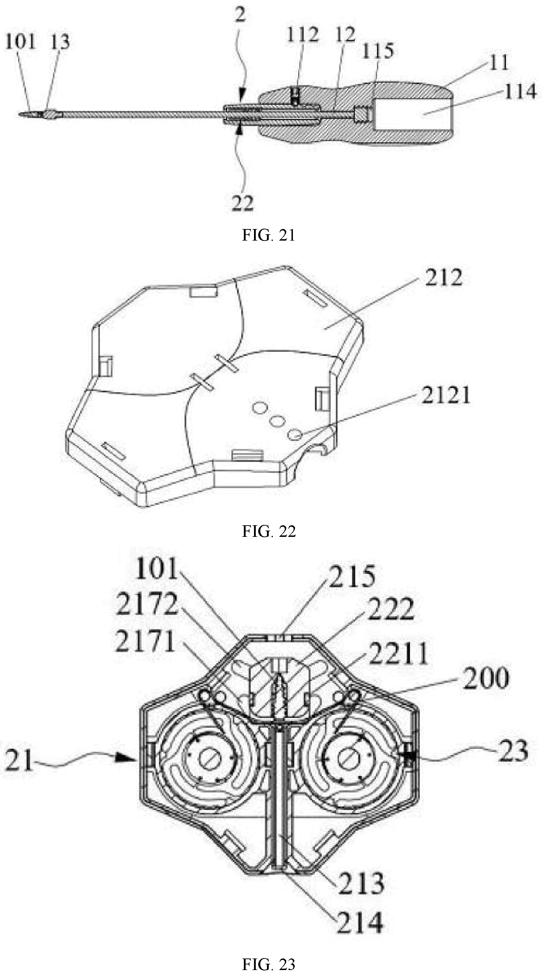

As shown in FIGS. 21 and 22, in order to ensure the engaging stability of the handle 11 and the storage box 2, the storage box 2 is provided with a spherical groove 2121, and a positioning bead 112 is provided on one side of the bayonet 111. The positioning bead 112 can cooperate with the spherical groove 2121 to fix the inserter 1 and the storage box 2. A through hole is provided on one side of the bayonet 111, and the positioning ball 112 is installed in the through hole. The positioning ball 112 is a ball plunger composed of a shell, a spring, and a ball or a cylinder. The ball retracts into the shell under pressure. When the ball is opposite to the spherical groove 2121, the ball stretches out to cooperate with the spherical groove 2121 under the elastic restoring force of the spring, so as to fix the storage box 2.

Specifically, the spherical groove 2121 is provided on the outer surface of the bottom shell 212, three spaced spherical grooves are designed along the center line of the bottom shell 212, and one positioning bead 112 is provided. The positioning bead 112 only needs to cooperate with one of the spherical grooves 2121 to fix the storage box 2.

The structural design of the storage box 2 provided in this embodiment converts simple insertion into programmed precise mechanical coordination. The insertion process completes mechanical operations, such as anchor extraction, release and tensioning of the suture 200, and anchor implantation under the coordination of the handle 11. The anchor clamping assembly 22 is designed as two symmetrical sliding clamping jaws 222 that cooperate with the insertion channel 213, and the clamping jaws 222 clamp the anchor by means of the elastic tape 2211. The ends of the two clamping jaws 222 near the inlet 214 are lifted by the insertion rod 12 pushing the anchor, and the other ends near the outlet 215 open to release the anchor. The release process is constrained by the elastic tape 2211, and the anchor clamping assembly 22 can be adapted to any type of anchor through simple structural improvement. The ratchets 23 and the guide damping columns 218 on two sides of the anchor clamping assembly 22 can semi-automatically manage the suture 200 and release the suture 200 of a set length according to demand. The guide damping columns 218 are used to ensure that the suture 200 is released from the ratchets 23 in a tensioned state. The suture 200, when stored in the box body 21, is "M"-shaped with a ring at the tail. The storage box 2 can achieve individual packaging of the anchor and cooperate with the reusable inserter 1 to achieve the assembly of the anchor and the inserter 1, with small volume, allowing for preoperative preparation of enough and various storage boxes 2 to ensure emergency response during surgery.

The inserter 1 provided in this embodiment is a reusable surgical tool, so its cost can be appropriately increased to enhance the strength of the structure and improve the stability of the mechanical structure. The insertion rod 12 of the inserter 1 can be quickly replaced, and the inserter is fixed with the storage box 2 through the positioning balls 112 on the handle 11. The insertion rod 12 and the insertion portion 13 are both made of hard stainless steel, thereby ensuring the quality of the insertion rod 12 and reducing the frequency of instrument failure during surgery due to quality problems of the inserter 1.

As shown in FIGS. 23 to 30, a usage method of the separable modular suture anchor implant system provided in this embodiment is as follows:
(1) One hand holds the handle 11 of the inserter 1, and the other hand holds the storage box 2.
(2) The insertion rod 12 enters the box body 21 from the inlet 214 of the storage box 2 and moves along the insertion channel 213. When the insertion portion 13 moves to align with the tail of the anchor, the insertion rod 12 is further pushed, and the insertion portion 13 is inserted into the anchor. Meanwhile, the insertion rod 12 and the anchor push the clamping jaws 222, the clamping jaws 222 move along the sliding tracks 217, and the two clamping jaws 222 overcome the constraint of the elastic tape 2211, so that the elastic tape 2211 undergoes elastic deformation. The ends of the two clamping jaws 222 near the outlet 215 open, and the anchor is pushed out from the accommodating chamber 223. During the movement of the anchor, the suture 200 drives the ratchets 23 to rotate through the guide damping columns 218 under tension, and the ratchets 23 begin to release the suture.
(3) The insertion rod 12 continues to move, the anchor is pushed forward and slides out of the box body 21 through the outlet 215, and the clamping jaws 222 do not continue to slide. Under the elastic restoring force of the elastic tape 2211, the two clamping jaws 222 move towards each other until they are in contact with the insertion rod 12 and the suture 200. The anchor drives the suture 200 to continue moving and drives the ratchets 23 to continue rotating.
(4) The insertion rod 12 pushes out the box body 21 forward, the ratchets 23 continue to rotate and release the suture, the insertion rod 12 continues to move until the box body 21 presses against the handle 11, and the positioning beads 112 at the bayonet 111 of the handle 11 cooperate with the spherical grooves 2121 to fix the relative positions of the box body 21 and the handle 11.
(5) The handle 11 is twisted to implant the anchor into the bone, and the suture 200 can be released.
(6) When the suture 200 is released, the head of the suture 200 is fixed at the tail of the anchor. The handle 11 is pulled in the direction opposite to the implantation direction, the box body 21 moves away from the anchor, the suture 200 drives the ratchets 23 to rotate, and the suture 200 is gradually released. During the release of the suture 200, the guide damping columns 218 maintain certain tension of the suture 200 in the pulling process.
(7) The inserter 1 continues to move in the direction opposite to the implantation direction, the ratchets 23 continue to rotate and release the suture until the tail end of the suture 200 breaks from the suture collection slots 2321 and slides out of the box body 21, and the release of the suture 200 is completed.
(8) After the release of the suture 200 is completed, the box body 21 is removed from the insertion rod 12.

After one anchor is implanted, when the next anchor is implanted, the above-mentioned inserter 1 is assembled with the next storage box 2, the implantation of the next anchor is continued according to the above steps, and so on until the implantation is completed.

### Second embodiment:

This embodiment provides a separable modular suture anchor implant system, which shares the same working principle and structural design of most components as the separable modular suture anchor implant system provided in the first embodiment. The overlapping details are omitted for brevity. The main differences lie in the structure of the insertion channel 213, the structure of the anchor clamping assembly 22, the suture storage and winding structure, and the structure for fixing the handle 11 and the storage box 2.

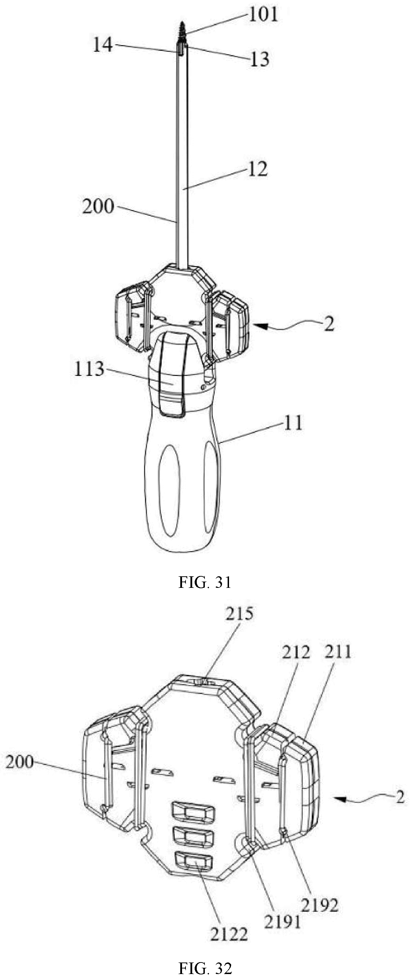

As shown in FIGS. 31 to 36, the insertion channel 213 is designed as a through type, with one end in communication with the inlet 214 and the other end in communication with the outlet 215. The insertion channel 213 extends from the inlet 214 to the outlet 215. The anchor clamping assembly 22 is provided on raised ribs, and the height of the raised ribs between the anchor clamping assembly 22 and the inlet 214 is higher than the height of the raised ribs below the anchor clamping assembly 22, to ensure that the center line of the anchor clamping assembly 22 and the center line of the insertion channel 213 are on the same straight line, the insertion rod 12 is inserted into the box body 21 and pushes out the anchor with higher stability, and the entire insertion rod 12 is constrained in the rotation direction during pushing.

Specifically, the height of the raised ribs between the anchor clamping assembly 22 on the bottom shell 212 and the inlet 214 is higher than the height of the raised ribs below the anchor clamping assembly 22. The raised ribs on the top shell 211 extend from the inlet 214 to the anchor clamping assembly 22.

Further, the connection between the inlet 214 and the insertion channel 213 is designed in a funnel shape, facilitating smooth entry of the insertion rod 12.

Specifically, the anchor clamping assembly 22 includes two clamping jaws 222, each clamping jaw 222 is provided with one sliding column 2222 on the same side, and the sliding track 217 extends away from the clamping jaw 222 and towards the outlet 215. Since the sliding column 2222 is provided on the same side of each clamping jaw 222, when the clamping jaw 222 moves, the sliding columns 2222 on two sides of the clamping jaw 222 are in sliding fit with the sliding tracks 217 on the top shell 211 and the bottom shell 212 respectively.

Further, the elastic member is provided around the sliding columns 2222 provided oppositely on the two clamping jaws 222. The sliding column 2222 includes a column body and a sliding head, the outer diameter of the sliding head is greater than that of the column body, the sliding head is slidably provided in the sliding track 217, the elastic member is an elastic rubber ring 2212, and the elastic rubber ring 2212 is snap-fitted between the sliding head and the side wall of the clamping jaw 222. Through such arrangement, the clamping jaws 222 can open a larger angle when releasing the anchor, thereby accommodating anchors of larger sizes. One elastic rubber ring 2212 may be provided, that is, one sides of the two clamping jaws 222 are constrained by the elastic rubber ring 2212; or two elastic rubber rings 2212 may be provided, that is, two sides of the two clamping jaws 222 are constrained by the elastic rubber rings 2212.

The suture storage and winding structure includes suture winding slots 2191 and suture collection holes 2192, the suture winding slots 2191 are symmetrically provided on the periphery of the storage box 2, one of the suture winding slots 2191 is provided with a suture passing hole 2193, and the suture collection holes 2192 are symmetrically provided on the periphery of the storage box 2 and are in communication with the interior of the storage box 2; the suture 200 passing through the anchor passes through the suture passing hole 2193 and is wound in the opposite suture winding slot 2191, and the tail end of the suture 200 is snap-fitted into the opposite suture collection hole 2192; and symmetrical lines of the suture winding slots 2191 and the suture collection holes 2192 are perpendicular to the center line of the insertion channel 213. Through such arrangement, the suture 200 passing through the anchor is threaded out of the box body 21 from the suture passing hole 2193, wound on the suture winding slots 2191 on the surface of the box body 21, and then fixed in the suture collection holes 2192 after being wound on the suture winding slots 2191 by several rounds. The suture 200 on the suture winding slots 2191 needs to be released manually, and the suture 200 cannot be automatically released when the insertion rod 12 pushes out the anchor.

Further, the suture storage and winding structure further includes a suture storage groove 2194, the suture storage groove 2194 is provided in the storage box 2, the suture 200 can be confined within the suture storage groove 2194, and the suture 200 passing through the anchor is led out through the suture storage groove 2194 and then passes through the suture passing hole 2193. The suture 200 enters the internal suture storage groove 2194 after passing through the anchor, is superposed by several rounds and then led out from the suture storage groove 2194, and passes through the suture passing hole 2193. This section stores the suture 200 inside the box body 21, and the suture storage groove 2194 extrudes and fixes the suture 200 stored inside the box body 21. When the anchor is pushed out, the suture 200 in the suture storage groove 2194 has certain resistance under the extrusion force.

Specifically, the suture storage groove 2194 is enclosed by three rectangular lugs provided at intervals between two opposite upright plates on the bottom shell 212 or the top shell 211, gaps are reserved between the two upright plates and two sides of the three rectangular lugs to form a U-shaped groove, limit plates are provided at upper ends of the rectangular lugs, the limit plate on the rectangular lug in the middle is connected to the first upright plate and spaced apart from the second upright plate, and the limit plates on the rectangular lugs on two sides are connected to the second upright plate and spaced apart from the first upright plate. The smaller spacing of the limit plates from the rectangular lugs and the upright plates is provided to compress the suture 200 and prevent the suture 200 from sliding out. The suture 200 is superposed by several turns in the U-shaped groove, then compressed by the limit plates, and led out from the U-shaped groove.

In other embodiments, the suture storage groove 2194 may be designed as a serpentine limit groove, and the suture 200 is stored in the serpentine limit groove.

Further, the length of the suture 200 stored in the box body 21 is equal to the length of the insertion rod 12. When the insertion rod 12 pushes out the anchor to an extreme position, the suture 200 in the suture storage groove 2194 is completely released.

Further, the suture storage and winding structure further includes a tensioning member 2195, the tensioning member 2195 is provided between the anchor and the suture storage groove 2194, and the suture 200 passing through the anchor is tensioned by the tensioning member 2195 and then enters the suture storage groove 2194. The tensioning member 2195 includes a guide upright plate and a guide upright column, the guide upright plate is provided on an outer side of the first upright plate and provided at an angle to the first upright plate, one end of the guide upright plate is connected to the first upright plate, and the other end is connected to the guide upright column. The suture 200 passing through the anchor bypasses the guide upright column and is guided into the suture storage groove 2194 via the guide upright plate, and the limit plates press the suture 200. When the suture 200 is released, the guide upright column and the guide upright plate can ensure that the suture 200 in the suture storage groove 2194 maintains certain tension when released.

In the inserter 1 provided in this embodiment, a snap-fitting member 113 is provided on one side of the bayonet 111 of the handle 11, and the snap-fitting member 113 is rotatably connected to the handle 11 and cooperates with the other side wall of the bayonet 111 to snap-fit with the storage box 2. By pressing one end of the snap-fitting member 113, the other end of the snap-fitting member 113 can move away from the other side wall of the bayonet 111. The clamping assembly includes a rocker clamping jaw, the rocker clamping jaw is rotatably connected to the handle 11 through a rotating shaft, and a pressing portion 1132 is provided at one end of the rocker clamping jaw. By pressing the pressing portion 1132, the other end of the rocker clamping jaw moves away from the other side wall of the bayonet 111 to release the storage box 2, and the storage box 2 is removed.

Specifically, the storage box 2 is provided with a plurality of snap-fitting grooves 2122, the other end of the snap-fitting member 113 is provided with a plurality of snap-fitting protrusions 1131, and the snap-fitting protrusions 1131 cooperate with the snap-fitting grooves 2122 for clamping. The snap-fitting protrusions 1131 are designed as trapezoidal teeth, thereby greatly increasing the connection strength between the storage box 2 and the handle 11, and preventing the storage box 2 from accidentally falling off. After use, the pressing portion 1132 is pressed to disassemble the storage box 2.

### Third embodiment:

This embodiment provides a separable modular suture anchor implant system, which shares the same working principle and structural design of most components as the separable modular suture anchor implant system provided in the first embodiment. The overlapping details are omitted for brevity. The main differences lie in the structure of the insertion portion 13 and the structure of the anchor clamping assembly 22.

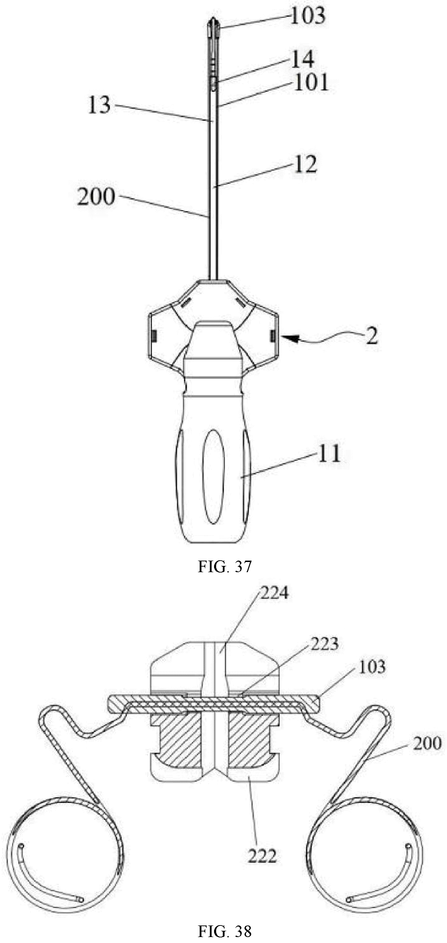

As shown in FIGS. 37 to 44, in the separable modular suture anchor implant system provided in this embodiment, the full suture anchor 103 is a flexible anchor that is flat in the middle, and the full suture anchor 103 is placed horizontally during storage; the insertion portion 13 includes an insertion connection rod 131 and a fork-shaped head 1311, and the fork-shaped head 1311 is provided at the end of the insertion connection rod 131 away from the insertion rod 12. After the fork-shaped head 1311 clamps the flat structure in the middle of the full suture anchor 103, the insertion rod 12 applies a force to the full suture anchor 103, and the full suture anchor 103 deforms and is V-shaped when being pushed out of the storage box 2. Improvements are made to the structure of the insertion portion 13 and the anchor clamping assembly 22 based on the structure of the full suture anchor 103.

In the anchor clamping assembly 22 provided in this embodiment, the center line of the accommodating chamber 223 is perpendicular to the symmetrical line of the two clamping jaws 222, the two clamping jaws 222 are connected to form a through hole 224 with a center line coinciding with the symmetrical line of the two clamping jaws 222, and the through hole 224 allows the insertion rod 12 to pass through. That is, the structures of the clamping jaws 222 are modified based on the structure of the full suture anchor 103, so that the storage box 2 is also suitable for storing the full suture anchor 103.

Further, in order to facilitate the pushing-out of the horizontally placed full suture anchor 103 from the anchor clamping assembly 22 by the insertion rod 12, the end of the clamping jaw 222 near the outlet 215 of the storage box 2 is designed as an opening 2221, and the accommodating chamber 223 is in communication with the opening 2221.

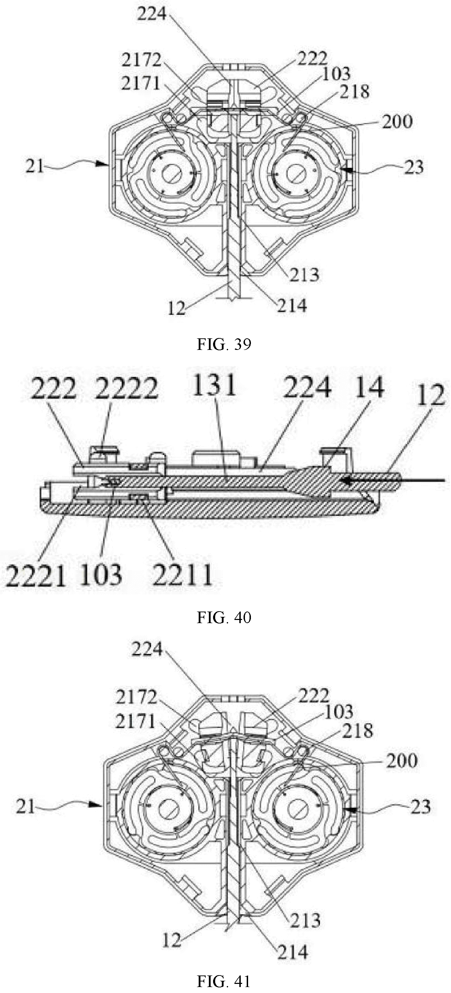

As shown in FIGS. 39 to 43, the full suture anchor 103 is horizontally placed in the accommodating chamber 223, and the suture 200 extends out from two ends of the full suture anchor 103 and is connected to the suture storage and winding structures on two sides; the insertion rod 12 enters the box body 21 via the through hole 224, moves until the fork-shaped head 1311 clamps the flat structure in the middle of the full suture anchor 103, and continues to move; the middle portion of the full suture anchor 103 deforms into a forward protrusion shape, and the clamping jaws 222 gradually open under the extrusion of the forward protrusion; the insertion rod 12 continues to move, and the full suture anchor 103 continues to move and is extruded into an approximate V shape; the clamping jaws 222 open a sufficient angle to allow the full suture anchor 103 to pass through, and then stop opening under the action of the elastic tape 2211; and the ratchets 23 begin to rotate under the traction of the suture 200. The insertion rod 12 continues to move, and the full suture anchor 103 can completely break from the opening 2221 and be pushed out of box body 21.

When the full suture anchor 103 is implanted, the tail of the handle 11 needs to be percussed to percuss the full suture anchor 103 into a predetermined position and depth, then the handle 11 held to pull out the insertion rod 12 in the direction opposite to the implantation direction, and the ratchets 23 rotate to release the suture 200. After the suture 200 is completely released, the implantation is completed, and the box body 21 is removed from the insertion rod 12.

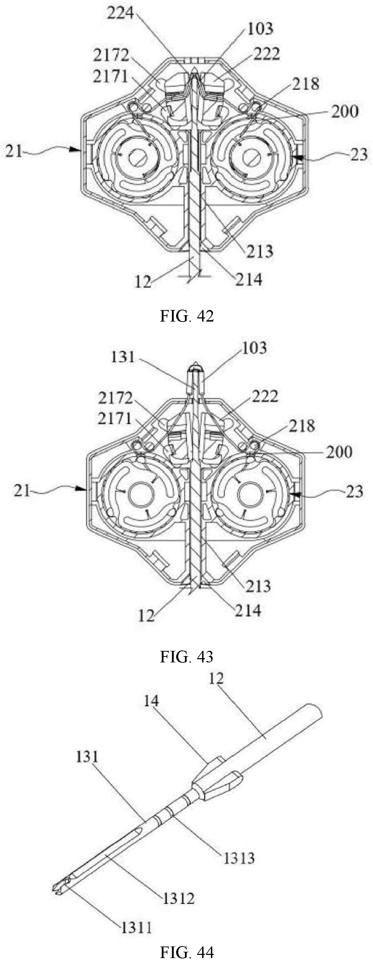

As shown in FIG. 44, the insertion connection rod 131 is further provided with a stress relief platform 1312 and scale lines 1313, and the scale lines 1313 are provided between the stress relief platform 1312 and the insertion rod 12. The stress relief platform 1312 functions to relieve extrusion contact stress when the implant is percussed. The scale lines 1313 indicate implantation depths during surgery. The diameter of the insertion connection rod 131 is smaller than that of the insertion rod 12, thereby reducing interface interference between the bone - the insertion connection rod 131 - anchor when the implant is percussed.

Described above are merely the preferred embodiments of the present application. For a person of ordinary skill in the art, modifications may be made to the specific implementation and application scope based on the idea of the present application, and the content of this specification should not be understood as limiting the present application.

## Claims

1. A separable modular suture anchor implant system, comprising:
an inserter (1), comprising an insertion rod (12), a head of the insertion rod (12) being provided with an insertion portion (13), and
a storage box (2), storing an anchor and a suture (200), two sides of the anchor being symmetrically provided with suture storage and winding structures, wherein
the suture (200) is configured to pass through the anchor and two ends of the suture (200) are connected to the suture storage and winding structures respectively, the suture storage and winding structures each comprises a ratchet (23) provided in the storage box (2) and comprising a central shaft (231), and the suture (200) is wound on the central shaft (231);
two ends of the storage box (2) are provided with an inlet (214) and an outlet (215) respectively, the insertion rod (12) is configured to insert into the storage box (2) via the inlet (214) so that the insertion portion (13) is inserted into the anchor, and the anchor is configured to be pushed out from the outlet (215) to complete the assembly of the inserter (1) and the anchor; and
after the anchor is implanted through the inserter (1), the insertion rod (12) is configured to drive the storage box (2) to move in a direction opposite to a implantation direction to disconnect the insertion portion (13) from the anchor, so that the suture storage and winding structures release the suture (200).

2. The separable modular suture anchor implant system according to claim 1, wherein the storage box (2) is provided with an anchor clamping assembly (22) comprising an elastic member and two symmetrically provided clamping jaws (222), the two clamping jaws (222) being connected through the elastic member to form an accommodating chamber (223), both the inlet (214) and the outlet (215) are in communication with the accommodating chamber (223), and the anchor is stored in the accommodating chamber (223); and
the two clamping jaws (222) are slidably provided in the storage box (2), under the push of the insertion rod (12), ends of the two clamping jaws (222) close to the outlet (215) slide and open away from each other to push the anchor out of the storage box (2).

3. The separable modular suture anchor implant system according to claim 2, wherein a center line of the accommodating chamber (223) coincides with a symmetrical line of the two clamping jaws (222); or
the center line of the accommodating chamber (223) is perpendicular to the symmetrical line of the two clamping jaws (222), the two clamping jaws (222) are connected to form a through hole (224) with a center line coinciding with the symmetrical line of the two clamping jaws (222), and the through hole (224) is configured to allow the insertion rod (12) to pass through.

4. The separable modular suture anchor implant system according to claim 2, wherein the storage box (2) is provided with a sliding track (217), and the clamping jaw (222) is provided with sliding columns (2222) in sliding connection with the sliding track (217);
each clamping jaw (222) is provided with the sliding columns (2222) at two ends on a same side, the sliding track (217) comprises a first sliding track (2171) and a second sliding track (2172), the first sliding track (2171) being provided close to the inlet (214), the second sliding track (2172) being provided close to the outlet (215), both the first sliding track (2171) and the second sliding track (2172) extending away from the clamping jaw (222) and towards the outlet (215), and the angle between the first sliding track (2171) and the symmetric line of the two clamping jaws (222) being smaller than the angle between the second sliding track (2172) and the symmetric line of the two clamping jaws (222); or
each clamping jaw (222) is provided with one sliding column (2222) on a same side, and the sliding track (217) extends away from the clamping jaw (222) and towards the outlet (215).

5. The separable modular suture anchor implant system according to claim 4, wherein a semi-annular groove (2223) is provided along the circumference of each of the clamping jaws (222), the semi-circular grooves (2223) of the two clamping jaws (222) are docked to form a mounting groove, in which the elastic member is provided; or
the elastic member is provided around the sliding columns (2222) provided oppositely on the two clamping jaws (222).

6. The separable modular suture anchor implant system according to claim 2, wherein the storage box (2) is further provided with an insertion channel (213), a center line of the anchor clamping assembly (22) and a center line of the insertion channel (213) are on a same straight line, one end of the insertion channel (213) is in communication with the inlet (214), and the other end of the insertion channel (213)is in communication with the accommodating chamber (223) or the outlet (215).

7. The separable modular suture anchor implant system according to claim 1, wherein the ratchet (23) comprises two baffles (232) provided at two ends of the central shaft (231), the baffle (232) is provided with suture collection slots (2321) that are spaced, and a tail end of the suture (200) is clamped into two adjacent ones of the suture collection slots (2321).

8. The separable modular suture anchor implant system according to claim 7, wherein a plurality of elastic pawls (233) are provided at intervals along the circumference of the baffle (232), annular grooves are formed between the elastic pawls (233) and the baffle (232), and one suture collection slot (2321) extending radially along the baffle (232) is provided inside each of the annular grooves.

9. The separable modular suture anchor implant system according to claim 7, wherein a guide damping column (218) is further provided on a peripheral side of the ratchet (23), and the suture (200) passing through the anchor bypasses the guide damping column (218) and is wound on the central shaft (231).

10. The separable modular suture anchor implant system according to claim 1, wherein the suture storage and winding structure comprise suture winding slots (2191) and suture collection holes (2192), the suture winding slots (2191) being symmetrically provided on the periphery of the storage box (2), one of the suture winding slots (2191) being provided with a suture passing hole (2193), and the suture collection holes (2192) being symmetrically provided on the periphery of the storage box (2) and are in communication with the interior of the storage box (2);
the suture (200) passing through the anchor is configured to pass through the suture passing hole (2193) and be wound in the opposite suture winding slot (2191), and a tail end of the suture (200) is clamped into the opposite suture collection hole (2192); and
symmetrical lines of the suture winding slots (2191) and the suture collection holes (2192) are perpendicular to a line connecting the inlet (214) and the outlet (215).

11. The separable modular suture anchor implant system according to claim 10, wherein the suture storage and winding structure further comprises a suture storage groove (2194) provided in the storage box (2), the suture (200) is confined within the suture storage groove (2194), and the suture (200) passing through the anchor is led out through the suture storage groove (2194) and then passes through the suture passing hole (2193).

12. The separable modular suture anchor implant system according to claim 11, wherein the suture storage and winding structure further comprises a tensioning member (2195) provided between the anchor and the suture storage groove (2194), and the suture (200) passing through the anchor is tensioned by the tensioning member (2195) and then enters the suture storage groove (2194).

13. The separable modular suture anchor implant system according to any one of claims 1 to 6, wherein the inserter (1) further comprises a handle (11), one end of the handle (11) is provided with a bayonet (111), the insertion rod (12) extends into the bayonet (111) and is detachably connected to the handle (11), and the bayonet (111) is configured to snap-fit with the storage box (2).

14. The separable modular suture anchor implant system according to claim 13, wherein the storage box (2) is provided with a spherical groove (2121), a positioning bead (112) is provided on one side of the bayonet (111), and the positioning bead (112) is configured to cooperate with the spherical groove (2121) to fix the inserter (1) and the storage box (2).

15. The separable modular suture anchor implant system according to claim 13, wherein one side of the bayonet (111) is provided with a snap-fitting member (113) rotatably connected to the handle (11) and is configured to cooperate with a wall of the other side of the bayonet (111) to snap-fit with the storage box (2); and
by pressing one end of the snap-fitting member (113), the other end of the snap-fitting member (113) moves away from the wall of the other side of the bayonet (111).

16. The separable modular suture anchor implant system according to claim 15, wherein the storage box (2) is provided with a plurality of snap-fitting grooves (2122), the other end of the snap-fitting member (113) is provided with a plurality of snap-fitting protrusions (1131) cooperating and snap-fitting with the plurality of snap-fitting grooves (2122).

17. The separable modular suture anchor implant system according to any one of claims 1 to 6, wherein the insertion portion (13) comprises a prismatic head, an internal hole head, or a fork-shaped head (1311).

18. The separable modular suture anchor implant system according to any one of claims 1 to 6, wherein the insertion portion (13) comprises an insertion connection rod (131), a diameter of the insertion connection rod (131) being smaller than that of the insertion rod (12), the insertion connection rod (131) being provided with a stress relief platform (1312) and scale lines (1313), the scale lines (1313) being provided between the stress relief platform (1312) and the insertion rod (12).

19. The separable modular suture anchor implant system according to any one of claims 1 to 6, wherein the insertion rod (12) is provided with a guide boss (14).

20. The separable modular suture anchor implant system according to any one of claims 1 to 6, wherein the storage box (2) comprises a box body (21), and the box body (21) comprises a top shell (211) and a bottom shell (212) that are snap-fitted.
